# FASCICULE DE BREVET EUROPEEN

(11) **EP 0 553 042 B1**
(45) Date de publication et mention de la délivrance du brevet: **01.04.1998**
(21) Numéro de dépôt: 93420024.7
(22) Date de dépôt: 21.01.1993
(51) Int. Cl.: A61B 17/58

(54) **Ensemble prothétique notamment pour l'articulation lombo-sacrée**
Stützgerät, insbesondere für das Lumbosakralgelenk
Support assembly, particularly for the lumbo-sacral joint

(30) Priorité: 23.01.1992 FR 9201042
(43) Date de publication de la demande: 28.07.1993
(73) Titulaire: EUROS Société Anonyme, F-13600 La Ciotat (FR); Gennari, Jean Marie, F-13008 Marseille (FR)
(72) Inventeur: Gennari, Jean Marie Les jardins de Thalassa-Bât. B, 13008 Marseille (FR)
(74) Mandataire: Dupuis, François

(56) Documents cités:
- EP-A- 0 301 489
- EP-A- 0 441 668
- US-A- 3 242 922
- US-A- 4 773 402
- US-A- 5 000 165

## Description

L'invention concerne plus particulièrement mais non limitativement, un ensemble prothétique, dans le cas d'une liaison dégénérative arthrosique pure de la charnière lombo-sacrée, nécessitant une arthrodèse entre la cinquième vertèbre lombaire et le sacrum.

A ce jour, les moyens utilisés pour de telles interventions, sont mal adaptés et limités en fonction de chaque cas pathologique à traiter.

Généralement, on utilise des plaques qui ne permettent pas d'assurer une fixation rationnelle et efficace au niveau de l'articulation entre les vertèbres lombaires et le sacrum.

Par le brevet EP 0441668 on connaît un ensemble prothétique pour l'articulation lombo-sacrée comprenant un élément destiné à être fixé au niveau de la région du sacrum et au niveau de la région lombaire, par l'intermédiaire d'un moyen rapporté. Toutefois, la solution décrite dans ce document ne donne pas satisfaction au niveau de la fixation de la région du sacrum et de la région lombaire, étant donné que cette fixation s'effectue au moyen d'une tige rapportée engagée dans des têtes de fixation. En outre, il n'y a pas de possibilité de réglage latéral entre deux ensembles prothétiques.

On connaît également par le brevet EP-A-0301489, une prothèse pour l'articulation lombo-sacrée destinée à solidariser le sacrum et les deux premières vertèbres lombaires (L4) et (L5). Elle se compose de deux éléments symétriques composés chacun d'une portion épousant une forme de tige solidairée au sacrum. Cette tige se prolonge vers le haut par une portion plate équipée de lumières ou de fentes pour permettre le passage de vis de fixation dans les vertèbres lombaires. Le fait que la plaque soit située au niveau de la région lombaire, va créer, avec les vis, une saillie sous cutanée étant donné qu'à ce niveau, l'épaisseur de chair est très réduite.

L'invention s'est fixée pour but de remédier à ces inconvénients, de manière simple, sûre efficace et rationnelle.

Le problème que se propose de résoudre l'invention est de réaliser une liaison particulièrement bien adaptée au niveau de la charniere lombo-sacrée.

Pour résoudre un tel problème, il a été conçu et mis au point un ensemble prothétique conforme à la revendication 1.

La plaque est formée angulairement par rapport à la tige.

Suivant une autre caractéristique, le moyen rapporté est conformé pour recevoir un organe de liaison apte à recevoir une tige d'accouplement pour réunir, avec capacité de réglage, au moins un autre élément.

Pour résoudre le problème posé d'assurer une fixation réglable, le moyen rapporté est constitué par une douille cylindrique montée avec capacité de déplacement en translation, le long de la tige de l'élément et dans un plan très sensiblement perpendiculaire, ladite douille recevant avec capacité d'orientation angulaire, une vis destinée à coopérer avec le pédicule.

L'extrémité de la douille où débouche la vis est biseautée pour pénétrer dans le pédicule.

Pour résoudre le problème posé d'assurer la liaison avec d'autres montages lombaires, la douille présente un évidement interne pour l'engagement, d'une part, de la tige de l'élément et, d'autre part, de l'organe de liaison, l'ensemble douille - organe de liaison étant bloqué en position sur la tige, par un bouchon vissé dans ladite douille.

Plus particulièrement, le problème posé d'assurer une liaison entre plusieurs éléments, est résolu en ce que l'organe de liaison présente une partie faisant office de berceau d'appui destinée à être engagée dans l'évidement de la douille, pour recevoir la tige de l'élément, ladite partie étant prolongée latéralement dans un plan orthogonal, par un manchon apte à recevoir à libre coulissement, la tige d'accouplement.

Dans une autre forme de réalisation, un organe obturateur est vissé dans la douille en étant conformé pour prendre appui sur la tige en vue de son blocage.

Cet organe obturateur comprend une bague présentant une échancrure diamétrale coopérant avec la section de la tige, ladite bague recevant à libre rotation, avec une position coaxiale fixe, un bouchon apte à être vissé dans l'alésage de la douille, pour assurer au fur et à mesure de son vissage, le déplacement de la bague en direction de la tige et provoquant de manière concomitante, son blocage sur l'effort de pression résultant.

Dans cette forme de réalisation, l'organe de liaison se présente sous forme d'un cavalier indépendant présentant une partie profilée faisant office de verseau d'appui, pour l'engagement de la tige, ledit organe présentant au moins un agencement apte à recevoir à libre coulissement, la tige d'accouplement.

Pour résoudre le problème posé d'assurer l'accouplement d'une manière simple et rapide, l'organe de liaison est bloqué en translation sur la tige au moyen d'écrous conformés pour assurer à volonté, soit le libre coulissement de la tige d'accouplement, soit son blocage en translation.

Avantageusement, l'écrou présente une tête polygonale et une portée cylindrique, coopérant avec une partie de l'organe de liaison, ladite portée étant fendue transversalement pour glisser le long de la tige sous l'effet d'une pince distractante.

L'invention est exposée, ci-après, plus en détail à l'aide des dessins annexés, dans lesquels :
La figure 1 est une vue en perspective montrant avant montage, les principaux éléments de l'ensemble prothétique selon une première forme de réalisation de l'invention.
La figure 2 est une vue en perspective correspondant à la figure 1, après montage des éléments constitutifs de l'ensemble.
La figure 3 est une vue en coupe considérée selon la ligne 3.3 de la figure 2.
La figure 4 est une vue en coupe considérée selon la ligne 4.4 de la figure 2.
La figure 5 est une vue en coupe de la douille.
La figure 6 est une vue en coupe du bouchon.
La figure 7 est une vue de la vis pédiculaire.
La figure 8 est une vue de l'une des vis sacrées.
La figure 9 est une vue en coupe longitudinale de la plaque tige.
La figure 10 est une vue en plan correspondant à la figure 9.
La figure 11 est une vue à caractère schématique montrant la mise en place de l'ensemble prothétique selon l'invention au niveau de l'articulation lombo-sacrée.
La figure 12 est une vue en perspective d'un organe obturateur dans une autre forme de réalisation de l'appareil.
La figure 13 est une vue en coupe de l'organe obturateur avant montage de la douille recevant la tige de l'élément prothétique.
La figure 14 est une vue correspondant à la figure 13 avant montage de l'organe obturateur dans la douille.
La figure 15 est une vue en perspective d'une autre forme de réalisation de l'organe de liaison.
La figure 16 est une vue en perspective de l'ensemble prothétique selon la forme de réalisation illustrée aux figures 12 à 15.

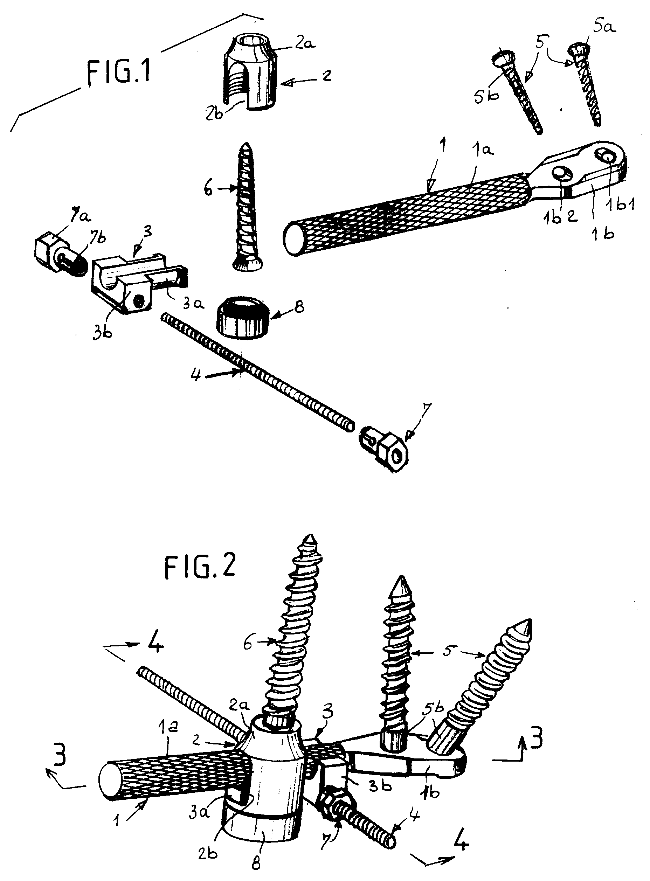

Comme le montre la figure 1, l'ensemble prothétique comprend un élément (1) présentant une partie (1b) apte à être fixée au niveau de la région du sacrum et une partie (1a) recevant au moins un moyen rapporté (2) pour sa fixation au niveau de la région lombaire.

Selon l'invention, la partie (1a) de l'élément (1) est constituée par une tige dont l'une des extrémités est évasée d'une manière progressive sous forme d'une plaque constituant la partie (1b). La plaque (1b) est formée angulairement par rapport à la tige (1a), en formant un angle (α) de l'ordre de 20°. Bien évidement, ce cintrage peut être modifié par l'opérateur, en fonction des courbures sagittales souhaitées. La tige (1a) présente une surface extérieure moletée.

La plaque (1b) est percée de deux orifices (1b1) (1b2) pour le passage de vis de fixation (5) destinées à être engagées au niveau de la région du sacrum. Les trous (1b1) et (1b2) sont orientés pour donner à chacune des vis (5), une orientation angulaire correspondante.

Le trou supérieur (1b1) est orienté intérieurement de très sensiblement 10° pour que la vis (5) correspondante, traverse le pédicule de la première vertèbre lombaire, pour finir sous le plateau du sacrum.

Le trou inférieur (1b2) est orienté extérieurement de très sensiblement 20° pour que la vis (5) qui le traverse, pénètre au dessus du premier trou sacré en direction de la corticale antérieure du sacrum.

Chacune des vis sacrée (5) présente au niveau de sa tête (5a) une portée cylindrique lisse (5b), pour diminuer les risques de rupture à la jonction vis-plaque.

A titre indicatif, la distance entre le trou (1b2) et la base de la plaque (1b), est de l'ordre de 16,3 mm. Or, l'écart moyen entre le premier trou sacré et le bord supérieur de l'aileron sacré est de l'ordre de 21 mm, ce qui laisse une marge de sécurité suffisante pour éviter de toucher la première racine sacrée.

A noter que la plaque (1b) présente, au niveau des trous (1b1) (1b2), un épaulement évitant tout risque de rupture à ce niveau. De même, il existe un élément (1) pour le côté droit et un élément (1) pour le côté gauche.

Le ou les moyens rapportés (2) sont constitués par une douille cylindrique montée avec capacité de déplacement en translation, le long de la tige (1a) de l'élément (1) et dans un plan très sensiblement perpendiculaire. Cette douille (2) reçoit coaxialement, avec capacité d'orientation angulaire, une vis (6) destinée à coopérer avec le pédicule, au niveau de la région lombaire. L'extrémité (2a) de la douille (2) où débouche la vis (6), est biseautée pour pénétrer dans le pédicule.

Il apparait donc que la distance entre la base de la tige (1a) et le pédicule, est la plus réduite possible. De même, après introduction de la vis (6) dans le pédicule, la douille (2) est orientable.

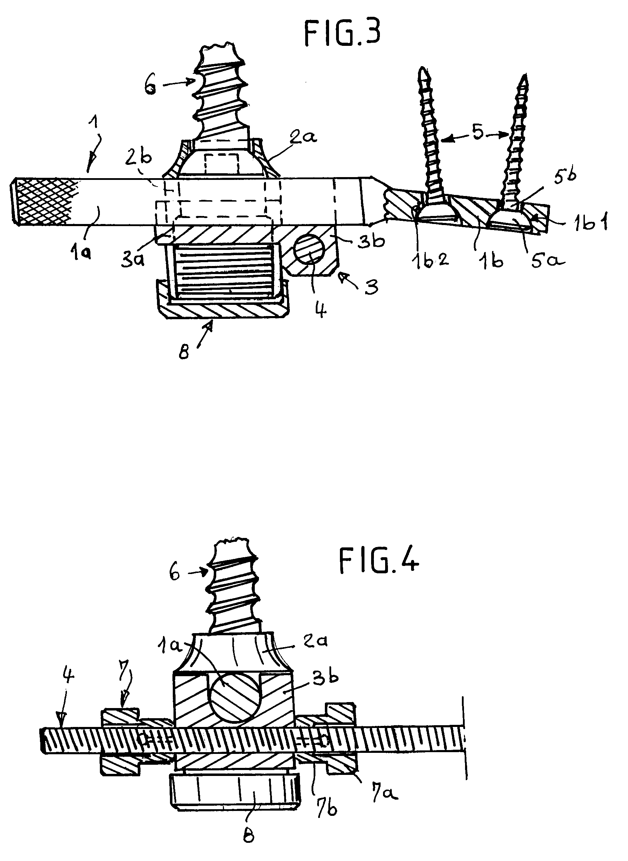

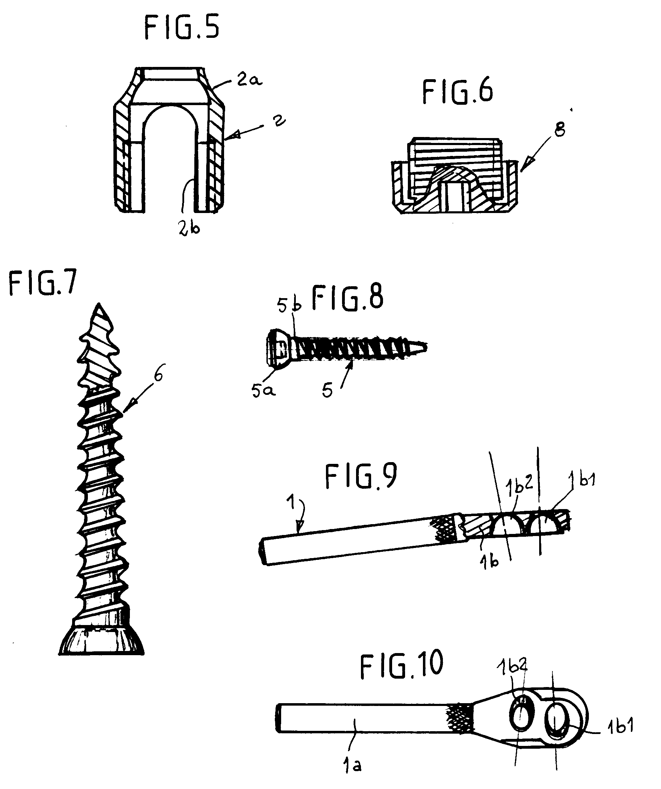

Dans la forme de réalisation illustrée aux figures 1 à 11, la douille (2) est conformée pour recevoir un organe de liaison (3) apte à recevoir une tige d'accouplement (4) pour unir, avec capacité de réglage, au moins un autre élément (1).

Le corps de la douille (2) présente un évidement interne (2b) formant un cavalier en U renversé pour l'engagement, d'une part, de la tige (1a) et, d'autre part, de l'organe de liaison (3). Dans ce but, l'organe de liaison présente une partie (3a) faisant office de berceau d'appui destinée à être engagée dans l'évidemment (2b) de la douille (2) pour recevoir la tige (1a) de l'élément (1). Ce berceau d'appui (3a) est prolongé latéralement, dans un plan orthogonal, par un manchon (3b) . Ce manchon est destiné à recevoir à libre coulissement, la tige d'accouplement (4) coopérant avec un autre manchon pour assurer l'accouplement de deux éléments (1). La tige (4) est filetée.

A noter que le blocage sur la tige (1a) de l'ensemble de la douille (2) équipée de l'organe de liaison (3), s'effectue par un bouchon (8) vissé dans ladite douille.

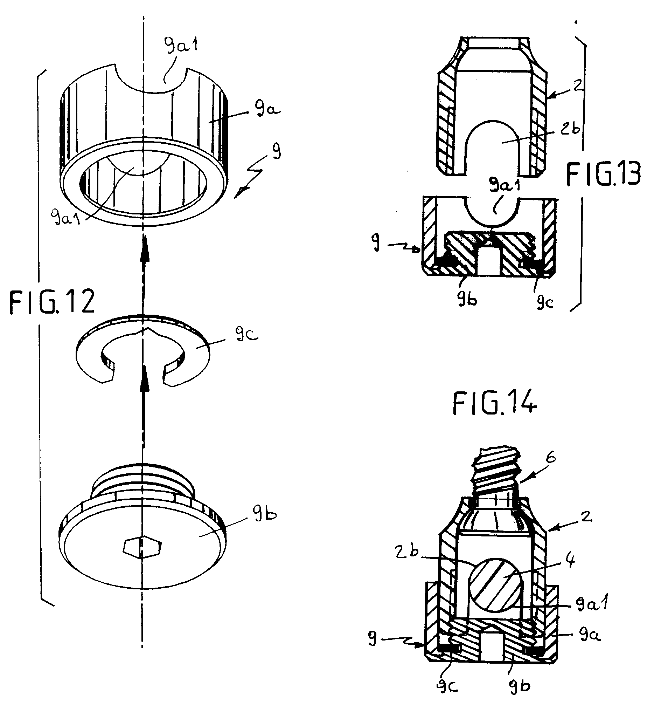

Dans la forme de réalisation illustrée aux figures 12 à 16, un organe obturateur (9) est vissé dans la douille (2) en étant conformé pour prendre appui sur la tige (1a) en vue de son blocage. Cet organe obturateur (9) comprend une bague (9a) présentant à l'une de ses extrémités, une échancrure diamétrale (9a1) coopérant avec la section de la tige (1a). A l'intérieur de cette bague est monté à libre rotation, un bouchon (9b) en appui sur le bord périphérique de ladite bague. Ce bouchon est immobilisé en translation au moyen d'un anneau ouvert (9c) engagé dans une gorge dudit bouchon et coopérant en appui avec un rebord interne de la bague. Ce bouchon (9b) est destiné à être vissé dans l'alésage de la douille (2), pour assurer d'une manière concomitante, le déplacement de la bague (9a) en direction de la tige. Ce déplacement a pour effet d'enserrer la section de la tige (1a) par l'effet conjugé de l'échancrure (9a1) de la bague et de l'évidement interne (2b) de la douille (2) provoquant ainsi le blocage de ladite tige (1a) sous l'effet du serrage en résultant.

Dans cette forme de réalisation, l'organe de liaison (3) se présente sous forme d'un cavalier indépendant. Ce cavalier présente une partie profilée (3c) faisant office de berceau d'appui pour l'engagement de la tige (1a). Ce cavalier (3) présente au moins un évidement (3d) pour l'engagement de la tige d'accouplement (4).

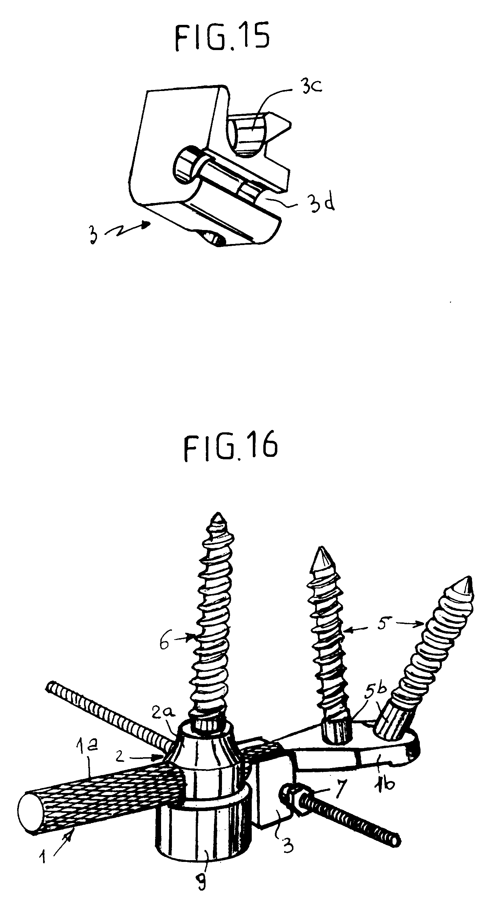

La figure 16 montre le montage de l'ensemble prothétique avec l'organe obturateur (9) et le cavalier de liaison (3) conforme à celui illustré figure 15.

Le blocage en translation des organes de liaison (3) sur la tige d'accouplement (4) s'effectue au moyen d'écrous (7) vissés sur ladite tige de part et d'autre de l'organe (3) au niveau du manchon (3b). Notamment, chacun des écrous est conformé pour être déplacé à libre coulissement sur la tige (4) puis vissé dans le manchon (3b) ou dans les évidements (3d).

L'écrou (7) présente une tête polygonale (7a) et une portée cylindrique (7b) destinée à être engagée dans le manchon (3b), ladite portée étant fendue transversalement pour glisser le long de la tige filetée sous l'effet d'une pince distractante. Ce n'est qu'après engagement dans le manchon (3b) de l'organe de liaison, que l'écrou est bloqué par un quart de tour.

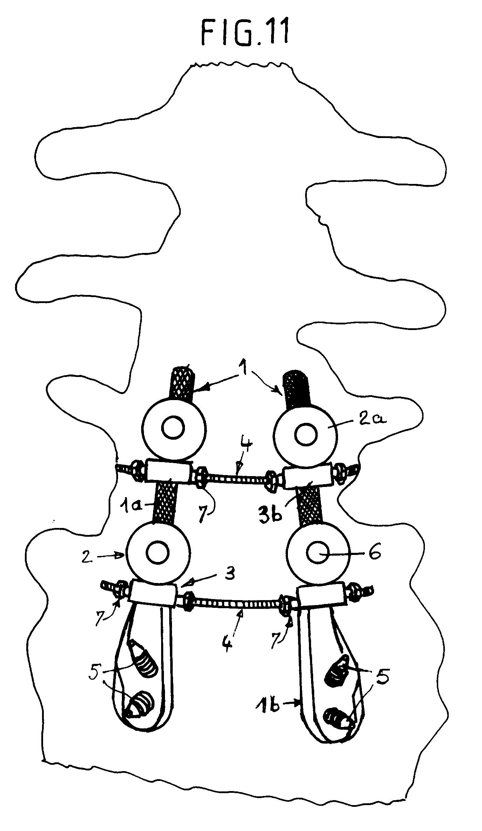

La figure 11 montre le montage de deux ensembles prothétiques selon l'invention, au niveau de l'articulation lombo-sacrée. En l'espèce, les deux éléments (1) sont fixés par leur plaque (1b) au niveau du sacrum, tandis que les tiges (1a) sont fixées au niveau de la région lombaire par les douilles (2) recevant la vis (6).

Dans l'exemple illustré, chacune des tiges reçoit deux ensembles douille (2) - organe de liaison (3), pour être accouplés par les tiges (4).

Les avantages ressortent bien de la description.

## Revendications

1. Ensemble prothétique notamment pour l'articulation lombo-sacrée, comprenant un élément (1) et un moyen rapporté (2), ledit élément (1) étant destiné à être fixé au niveau de la région du sacrum et au niveau de la région lombaire, par l'intermédiaire du moyen rapporté (2), chaque élément (1) présentant une tige (1a) dont l'une des extrémités est évasée sous forme d'une plaque (1b), caractérisé en ce que :
- ladite plaque (1b) est équipée de deux trous orientés (1b1) (1b2) pour le passage de vis de fixation (5),
- le trou (1b1), situé à l'extrémité libre de la plaque (1b), étant orienté intérieurement de très sensiblement 10°, pour que la vis (5) traverse le pédicule de la première vertèbre et finisse sous le plateau du sacrum,
- l'autre trou (1b2) est orienté extérieurement de très sensiblement 20°, pour que la vis (5) qui le traverse, pénètre au dessus du premier trou sacré en direction de la corticale antérieure du sacrum.

2. Ensemble prothétique selon la revendication 1, caractérisé en ce que la plaque (1b) est formée angulairement par rapport à la tige (1a).

3. Ensemble prothétique selon la revendication 1, caractérisé en ce que le moyen (2) est conformé pour recevoir un organe de liaison (3) apte à recevoir une tige d'accouplement (4) pour réunir, avec capacité de réglage, au moins un autre élément (1).

4. Ensemble prothétique selon la revendication 1, caractérisé en ce que le moyen rapporté (2) est constitué par une douille cylindrique montée avec capacité de déplacement en translation, le long de la tige (1a) de l'élément (1) et dans un plan très sensiblement perpendiculaire, ladite douille (2) recevant avec capacité d'orientation angulaire, une vis (6) destinée à coopérer avec le pédicule.

5. Ensemble prothétique selon la revendication 4, caractérisé en ce que l'extrémité de la douille (2) où débouche la vis (6) est biseautée pour pénétrer dans le pédicule.

6. Ensemble prothétique selon la revendication 4, caractérisé en ce que la douille (2) présente un évidement interne (2b) pour l'engagement, d'une part, de la tige (1a) de l'élément (1) et, d'autre part, de l'organe de liaison (3), l'ensemble douille (2) - organe de liaison (3) étant bloqué en position sur la tige (1a), par un bouchon vissé (8) dans ladite douille (2).

7. Ensemble prothétique selon la revendication 1, caractérisé en ce qu'un organe obturateur (9) est vissé dans la douille (2) en étant conformé pour prendre appui sur la tige (1a) en vue de son blocage.

8. Ensemble prothétique selon la revendication 7, caractérisé en ce que l'organe obturateur (9) comprend une bague (9a) présentant une échancrure diamétrale (9a1) coopérant avec la section de la tige (1a), ladite bague recevant à libre rotation, avec une position coaxiale fixe, un bouchon (9b) apte à être vissé dans l'alésage de la douille (2), pour assurer au fur et à mesure de son vissage, le déplacement de la bague (9a) en direction de la tige (1a) et provoquant de manière concomitante, son blocage sur l'effort de pression résultant.

9. Ensemble prothétique selon la revendication 3, caractérisé en ce que l'organe de liaison (3) présente une partie (3a) faisant office de berceau d'appui destinée à être engagée dans l'évidement (2b) de la douille (2), pour recevoir la tige (1a) de l'élément (1), ladite partie (3a) étant prolongée latéralement dans un plan orthogonal, par un manchon (3b) apte à recevoir à libre coulissement, la tige d'accouplement (4).

10. Ensemble prothétique selon la revendication 3, caractérisé en ce que l'organe de liaison (3) se présente sous forme d'un cavalier indépendant présentant une partie profilée (3c) faisant office de verseau d'appui, pour l'engagement de la tige (1a), ledit organe présentant au moins un agencement (3d) apte à recevoir à libre coulissement, la tige d'accouplement (4).

11. Ensemble prothétique selon l'une quelconque des revendications 9 et 10, caractérisé en ce que l'organe de liaison (3) est bloqué en translation sur la tige (4) au moyen d'écrous (7) conformés pour assurer à volonté, soit le libre coulissement de la tige d'accouplement (4), soit son blocage en translation.

12. Ensemble prothétique selon la revendication 11, caractérisé en ce que l'écrou (7) présente une tête polygonale (7a) et une portée cylindrique (7b), coopérant avec le manchon (3b) de l'organe de liaison (3), ladite portée (7b) étant fendue transversalement pour glisser le long de la tige (4) sous l'effet d'une pince distractante.

## Claims

1. Prothesis assembly, especially for the lumbo-sacral joint, comprising an element (1) and a separately mounted means (2), said element (1) being designed to be fixed at the level of the region of the sacrum and at the level of the lumbar region through separately mounted means (2), each element (1) having a rod (1a) of which one of the ends is flattened in the form of a plate (1b) characterised in that:
- said plate (1b) has two slanting holes (1b1) (1b2) through which a fixing screw (5) passes,
- hole (1b1) situated at the free end of plate (1b) slants internally at substantially 10° so that screw (5) passes through the pedicle of the first vertebra and finishes under the disc of the sacrum,
- the other hole (1b2) slants externally at substantially 20° so that screw (5) that passes through it penetrates above the first sacral hole in the direction of the anterior cortical of the sacrum.

2. Prothesis assembly as claimed in claim 1 characterised in that plate (1b) is angularly shaped with respect to rod (1a).

3. Prothesis assembly as claimed in claim 1 characterised in that means (2) is shaped to accommodate a coupling (3) capable of accommodating a connecting rod (4) in order to join at least one other element (1) with the capacity for adjustment.

4. Prothesis assembly as claimed in claim 1 characterised in that the separately mounted means (2) consists of a cylindrical bush mounted with the capacity for translational movement along rod (1a) of element (1) in a plane that is substantially perpendicular, said bush (2) accommodating a screw (6) capable of being angularly swivelled and intended to cooperate with the pedicle

5. Prothesis assembly as claimed in claim 4 characterised in that the end of bush (2) where screw (6) emerges is bevelled in order to penetrate into the pedicle.

6. Prothesis assembly as claimed in claim 4 characterised in that bush (2) has an internal cavity (2b) in order to fit, on the one hand, the rod (1a) of element (1) and, on the other hand, the coupling (3), with the bush (2)/coupling (3) assembly being locked in position on rod (1a) by a plug (8) that is screwed into said bush (2).

7. Prothesis assembly as claimed in claim 1 characterised in that a blanking device (9) is screwed into bush (2) and is shaped in order to rest on rod (1a) so that it can be locked.

8. Prothesis assembly as claimed in claim 7 characterised in that the blanking device (9) comprises a ring (9a) having a cut-out around its diameter (9a1) that cooperates with the cross section of rod (1a), said ring accommodating a freely rotating plug (9b) with a fixed coaxial position capable of being screwed into the bore of bush (2) in order to ensure, as it is gradually screwed up, displacement of ring (9a) in the direction of rod (1a), thereby causing its locking by the resulting pressure force.

9. Prothesis assembly as claimed in claim 3 characterised in that the coupling (3) has a part (3a) that acts as a support cradle intended to be fitted in the cavity (2b) of bush (2) in order to accommodate rod (1a) of element (1), said part (3a) being laterally extended in an orthogonal plane by a sleeve (3b) capable of accommodating the freely sliding connecting rod (4).

10. Prothesis assembly as claimed in claim 3 characterised in that the coupling (3) is in the shape of an independent carrier having a profiled part (3c) acting as a support cradle in order to fit rod (1a), said device having at least one feature (3d) capable of accommodating the freely sliding connecting rod (4).

11. Prothesis assembly as claimed in claim 9 and 10 characterised in that translational movement of coupling (3) is prevented on rod (4) by means of nuts (7) shaped in order to deliberately ensure either free sliding of connecting rod (4) or prevention of translational movement of the connecting rod.

12. Prothesis assembly as claimed in claim 11 characterised in that nut (7) has a polygonal head (7a) and a cylindrical bearing surface (7b) that cooperates with sleeve (3b) of coupling (3), said bearing surface (7b) being transversely split in order to slide along rod (4) under the effect of a spreader.

## Patentansprüche

1. Prothesensystem insbesondere für das Lumbalsakralgelenk bestehend aus einem Element (1) und einer Aufsetzvorrichtung (2), wobei das besagte Element (1) zur Befestigung im Bereich der Kreuzbein- und Lendenwirbelregion mit Hilfe der Aufsetzvorrichtung (2) bestimmt ist, wobei jedes Element (1) einen Schaft (1a) aufweist, von dem ein Endstück in Form einer Platte (1b) verbreitert ist, dadurch gekennzeichnet, daß:
- diese Platte (1b) zwei gerichtete Bohrungen (1b1) (1b2) für die Durchführung von Befestigungsschrauben (5) aufweist,
- die Bohrung (1b1) am freien Ende der Platte (1b) innen um ziemlich genau 10° ausgerichtet ist, damit die Schraube (5) den Hilus des ersten Wirbels durchquert und unter dem Kreuzbeinplateau austritt,
- die andere Bohrung (1b2) außen um ziemlich genau 20° ausgerichtet ist, damit die sie durchquerende Schraube (5) oberhalb des ersten Kreuzbeinlochs in Richtung vordere Kreuzbeinrinde verläuft.

2. Prothesensystem nach Anspruch 1, dadurch gekennzeichnet, daß die Platte (1b) im Verhältnis zum Schaft (1a) winkelig ausgebildet ist.

3. Prothesensystem nach Anspruch 1, dadurch gekennzeichnet, daß die Vorrichtung (2) dafür ausgelegt ist, ein Verbindungsorgan (3) aufzunehmen, das geeignet ist, eine Kupplungsstange (4) aufzunehmen, um mindestens ein anderes verstellbares Element (1) zu bilden.

4. Prothesensystem nach Anspruch 1, dadurch gekennzeichnet, daß die Aufsetzvorrichtung (2) aus einer zylindrischen Hülse besteht, die in der Translation auf dem Schaft (1a) des Elements (1) und auf einer ziemlich genau senkrechten Ebene verschiebbar angebracht ist, wobei die Hülse (2) eine winkelig ausrichtbare Schraube (6) aufnimmt, die mit dem Hilus zusammenwirken soll.

5. Prothesensystem nach Anspruch 4, dadurch gekennzeichnet, daß das Ende der Hülse (2), an dem die Schraube (6) austritt, zum besseren Eindringen in den Hilus abgefast ist.

6. Prothesensystem nach Anspruch 4, dadurch gekennzeichnet, daß die Hülse (2) eine innere Aussparung (2b) zum Einführen einerseits des Schaftes (1a) des Elements (1) und andererseits des Verbindungsorgans (3) aufweist, wobei das Teilsystem aus Hülse (2) und Verbindungsorgan (3) durch einen in die Hülse (2) eingeschraubten Verschluß (8) auf dem Schaft (1a) in einer bestimmten Stellung blockiert wird.

7. Prothesensystem nach Anspruch 1, dadurch gekennzeichnet, daß in die Hülse (2) ein Verschlußorgan (9) eingeschraubt wird, das so ausgebildet ist, um zu dessen Fixierung am Schaft (1a) anzusetzen.

8. Prothesensystem nach Anspruch 7, dadurch gekennzeichnet, daß das Verschlußorgan (9) einen Ring (9a) mit einer diametralen Auskerbung (9a1) aufweist, die mit dem Querschnitt des Schaftes (1a) zusammenwirkt, wobei dieser Ring bei unbehinderter Drehung mit fester koaxialer Stellung einen Verschluß (9b) aufnimmt, der in die Bohrung der Hülse (2) eingeschraubt werden kann, um nach Maßgabe des Einschraubens das Verschieben des Ringes (9a) in Richtung des Schaftes (1a) zu gewährleisten und aufgrund der daraus resultierenden Druckkraft seine Fixierung zu bewirken.

9. Prothesensystem nach Anspruch 3, dadurch gekennzeichnet, daß das Verbindungsorgan (3) einen Teil (3a) aufweist, der als Auflager dient und dazu bestimmt ist, in die Aussparung (2b) der Hülse (2) eingeführt zu werden, um den Schaft (1a) des Elements (1) aufzunehmen, wobei der Teil (3a) seitlich in orthogonaler Ebene durch eine Muffe (3b) verlängert wird, die geeignet ist, die Kupplungsstange (4) frei gleitend aufzunehmen.

10. Prothesensystem nach Anspruch 3, dadurch gekennzeichnet, daß das Verbindungsorgan (3) die Form eines selbständigen Reiters mit einem profilierten Teil (3c) als Auflager für die Einführung des Schaftes (1a) aufweist, wobei das Organ mindestens eine Vorkehrung (3d) aufweist, die geeignet ist, die Kupplungsstange (4) frei gleitend aufzunehmen.

11. Prothesensystem nach Anspruch 9 oder 10, dadurch gekennzeichnet, daß das Verbindungsorgan (3) in der Translation auf dem Schaft (4) mit Muttern (7) fixiert wird, die so ausgebildet sind, um beliebig entweder für das freie Gleiten der Kupplungsstange (4) oder ihre Fixierung in Translationsrichtung zu sorgen.

12. Prothesensystem nach Anspruch 11, dadurch gekennzeichnet, daß die Mutter (7) einen polygonalen Kopf (7a) und eine zylindrische Tragfläche (7b) aufweist, die mit der Muffe (3b) des Verbindungsorgans (3) zusammenwirkt, wobei diese Tragfläche (7b) querverlaufend gespalten ist, um unter Einwirkung einer Spreizklammer am Schaft (4) entlangzugleiten.
